# FASCICULE DE BREVET EUROPEEN

(11) **EP 1 073 673 B1**
(45) Date de publication et mention de la délivrance du brevet: **10.01.2007**
(21) Numéro de dépôt: 99915834.8
(22) Date de dépôt: 26.04.1999
(51) Int. Cl.: C07K 5/06

(54) **DERIVES DE L'ACIDE OCTAHYDRO-6, 10-DIOXO-6H-PYRIDAZINO/1,2-A/ /1,2/ DIAZEPINE-1-CARBOXYLIQUE, LEUR PROCEDE DE PREPARATION ET LEUR APPLICATION A LA PREPARATION DE COMPOSES THERAPEUTIQUEMENT ACTIFS**
OCTAHYDRO-6, 10-DIOXO-6H-PYRIDAZINO/1,2-A/ /1,2/ DIAZEPIN-1-CARBONSÄURE-DERIVATE, VERFAHREN ZU DEREN HERSTELLUNG UND DEREN VERWENDUNG ZUR HERSTELLUNG PHARMAZEUTISCHER WIRKSTOFFE
OCTAHYDRO-6,10-DIOXO-6H-PYRIDAZINO/1,2-a/ /1,2/DIAZEPIN-1-CARBOXYLIC ACID DERIVATIVES, PREPARATION METHOD AND USE FOR PREPARING THERAPEUTICALLY ACTIVE COMPOUNDS

(30) Priorité: 27.04.1998 FR 9805243
(43) Date de publication de la demande: 07.02.2001
(73) Titulaire: Aventis Pharma S.A., 92160 Antony (FR)
(72) Inventeur: COLLADANT, Colette, F-93110 Rosny sous Bois (FR); CROCQ, Véronique, F-21000 Dijon (FR); LARKIN, John, Patrick, F-78600 Maisons Lafitte (FR); ROUSSEL, Patrick, F-94320 Thiais (FR)
(86) Numéro de dépôt international: PCT/FR1999/000981
(87) Numéro de publication internationale: WO 1999/055724

(56) Documents cités:
- WO-A-93/23403
- WO-A-95/33751
- WO-A-97/22619
- US-A- 4 512 924
- US-A- 5 656 627
- US-A- 5 723 602

## Description

La présente invention concerne de nouveaux dérivés de l'acide octahydro-6,10-dioxo-6H-pyridazino[1,2-a][1,2] diazépine-1-carboxylique, leur procédé de préparation et leur application à la préparation de composés thérapeutiquement actifs.

Le brevet US4512924 se rapporte à des pyridazo[1,2-a][1,2]diazépines substitués en position 9 par un groupe aminoalkyle susbtitué. La demande de brevet WO 93/23403 et le brevet US5723602 se rapportent à des dérivés mercaptoacetylamide de pyridazo[1,2]pyridazines, pyrazolo[1,2]pyridazines, pyridazo[1,2-a][1,2]diazépines. Les demandes de brevet WO 97/22619, WO 95/33751 et le brevet US 5656627 se rapportent à des dérivés pyridazino[1,2-a][1,2]diazépines substitués en position 1 par un groupe carbonylamino.

L'invention a pour objet les composés de formule (I) : 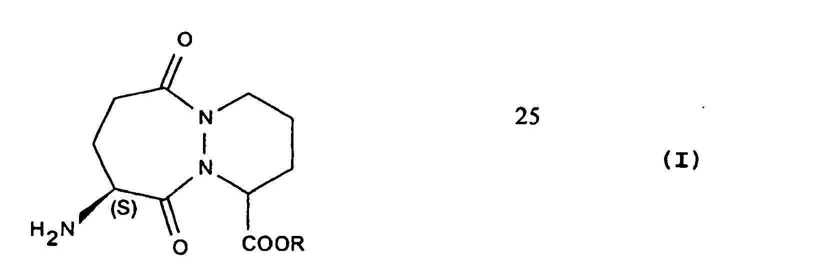 sous forme de mélange 9S1R+9S1S, dans laquelle R représente un atome d'hydrogène, un radical alkyle ou aralkyle renfermant jusqu'à 18 atomes de carbone, la fonction amine pouvant être libre ou protégée.

R représente par exemple un radical H, méthyle, éthyle, propyle, isopropyle, n-butyle, isobutyle ou tertbutyle, ou un radical benzyle ou naphtyle. Lorsque la fonction amine est protégée, la protection peut se faire selon les méthodes classiques de protection des amines.

L'invention a notamment pour objet les composés répondant à la formule (IA) : 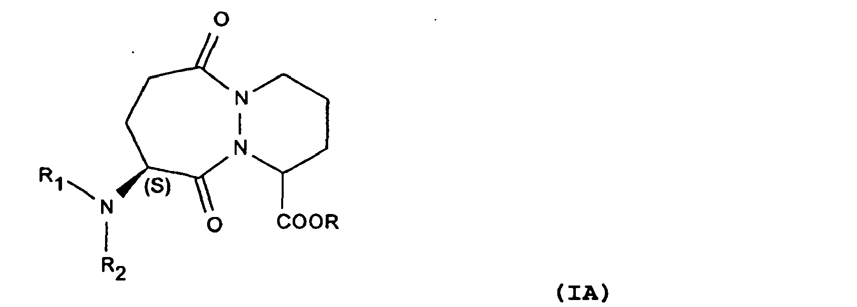 sous forme de mélange 9S1R+9S1S, dans laquelle R conserve sa signification précédente et/ou bien R₁ représente un radical : 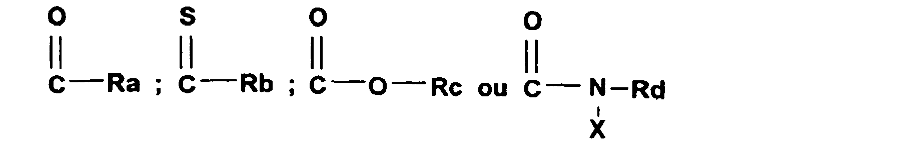 Ra, Rb, Rc et Rd représentant un radical alkyle ou aryle renfermant jusqu'à 18 atomes de carbone, ou un radical mono-, bi- ou tricyclique renfermant un ou plusieurs hétéroatomes,
X représentant un atome d'hydrogène, un radical alkyle renfermant jusqu'à 8 atomes de carbone ou un radical aryle renfermant jusqu'à 14 atomes de carbone, et R₂ représente un atome d'hydrogène, ou bien R₁ et R₂ forment ensemble un radical mono ou polycyclique renfermant un ou plusieurs hétéroatomes.
On peut par exemple utiliser pour protéger les amines, des composés cycliques par exemple les radicaux : 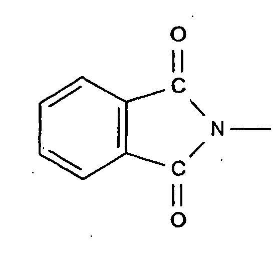 ou encore le radical : 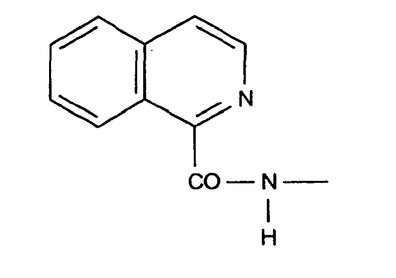 L'invention a plus particulièrement pour objet les composés de formule (IA) dans lesquels R₁ et R₂ forment ensemble un radical polycyclique renfermant un ou plusieurs hétéroatomes et notamment les composés répondant à la formule (IA₁) : 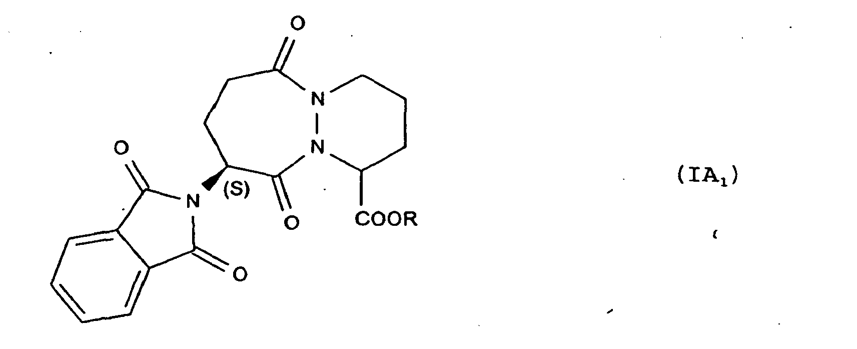 sous forme de mélange 9S1R+9S1S

L'invention a notamment pour objet les composés de formule (I) dans laquelle R représente un radical méthyle, sous forme de mélange 9S1R-9S1S.

L'invention a également pour objet un procédé caractérisé en ce que l'on soumet un composé de formule (II) : 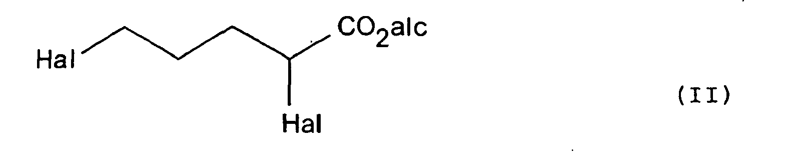 dans laquelle alc représente un radical alkyle renfermant jusqu'à 8 atomes de carbone et Hal représente un atome d'halogène, à l'action d'un composé de formule (III) : 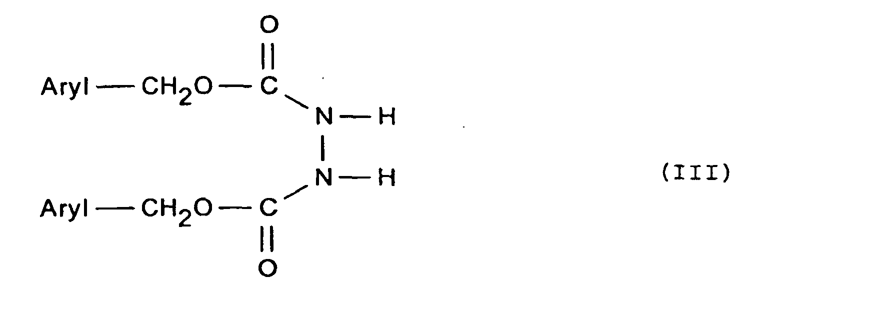 dans laquelle Aryl représente un radical aryle renfermant jusqu'à 14 atomes de carbone, pour obtenir le composé de formule (IV) : 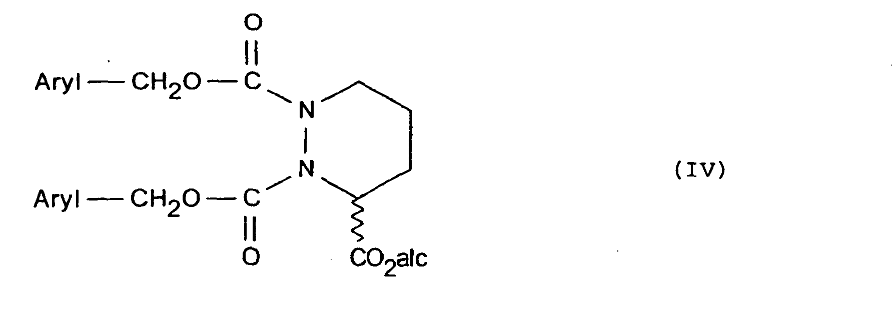 que l'on soumet à l'action d'un agent basique, pour obtenir le composé de formule (V) : 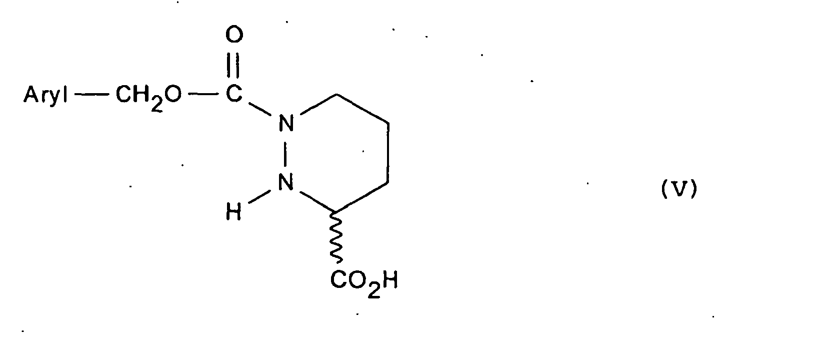 que l'on soumet éventuellement à l'action d'un agent d'alkylation pour obtenir le composé de formule (VI) : 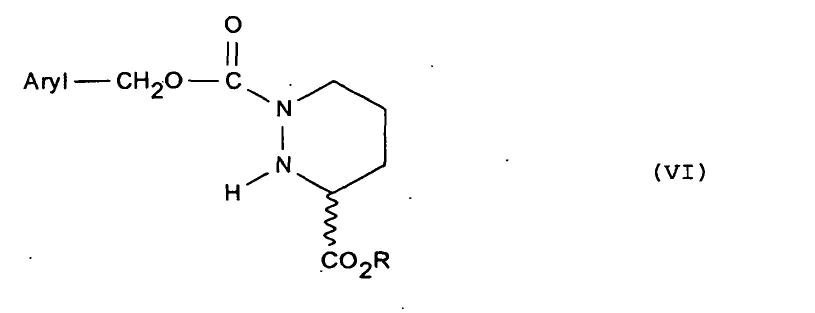 que l'on soumet à l'action d'un composé de formule (VII) : 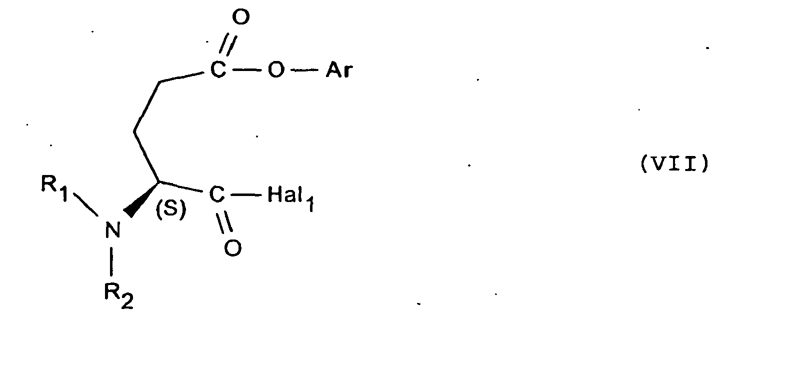 dans laquelle Hal₁ représente un atome d'halogène et Ar représente un radical aryle ou aralkyle renfermant jusqu'à 18 atomes de carbone, R₁ et R₂ conservant la même définition que précédemment, pour obtenir le composé de formule (VIII) : 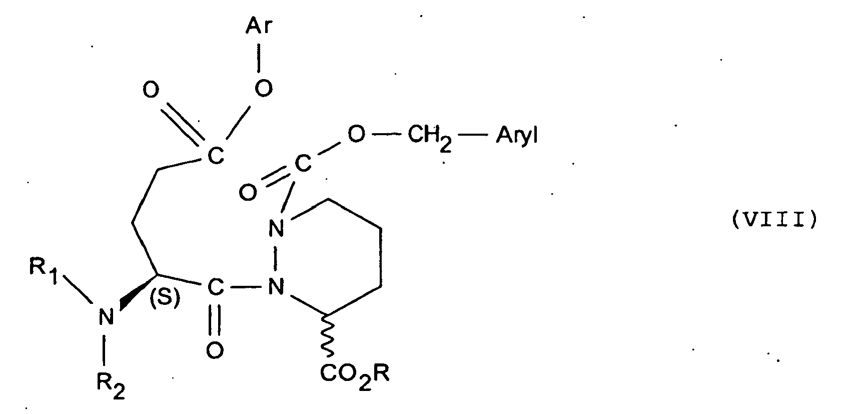 sous forme de mélange 9S1R+9S1S, que l'on soumet à l'action d'un agent d'hydrogénation pour obtenir le composé de formule (IX) : 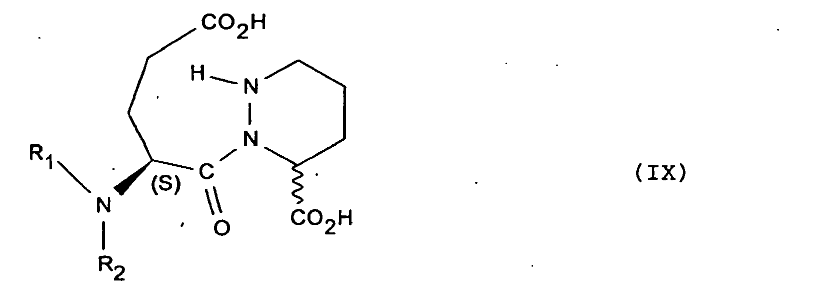 sous forme de mélange 9S1R+9S1S, que l'on soumet à l'action d'un agent de condensation pour obtenir le composé de formule (IA) correspondant, puis si désiré, libère la fonction amine pour obtenir le composé de formule (I) dans lequel la fonction amine est libre.

Dans un mode de réalisation préféré :
- Hal et Hall représentent un atome de chlore,
- alc représente un radical alcoyle renfermant jusqu'à 4 atomes de carbone,
- Aryl représente un radical phényle, ou naphtyle
- aralkyle représente un radical benzyle,
- la réaction entre les composés de formule (II) et de formule (III) a lieu en présence d'une base par exemple en présence d'un carbonate alcalin comme le carbonate de potassium,
- l'agent basique que l'on fait réagir sur le composé de formule (IV) est l'hydroxyde de sodium ou de potassium,
- l'agent d'alkylation que l'on fait réagir sur le composé de formule (V) est un alcool par exemple le méthanol,
- la condensation entre les composés (VI) et (VII) est réalisée en présence d'une base comme la pyridine, la TEA, la diisopropylamine,
- l'agent d'hydrogénation est par exemple l'hydrogène en présence de palladium sur charbon, de dihydroxyde de palladium en présence de talc, de rhodium en présence d'alumine, de ruthénium sur charbon, ou en présence de nickel de Raney,
- la cyclisation est réalisée en présence de SOCl₂ ou PCl₅ ou d'esters activés ou en présence d'agents déshydratants comme l'APTS,
- la libération de l'amine peut être réalisée au moyen de l'hydrazine.

Les produits (IV), (VIII) et (IX) mis en oeuvre au cours du procédé sont des produits nouveaux et sont en eux-mêmes un objet de la présente invention.

L'invention a plus particulièrement pour objet les produits dont la préparation est donnée ci-après dans la partie expérimentale et notamment le mélange racémique.

L'invention a également pour objet l'application caractérisée en ce que l'on soumet un composé de formule (I) sous forme de mélange 9S1R+9S1S, à l'action d'un agent de déracémisation du carbone asymétrique porté par le cycle en position 6, à la préparation du composé de formule (Iopt) : 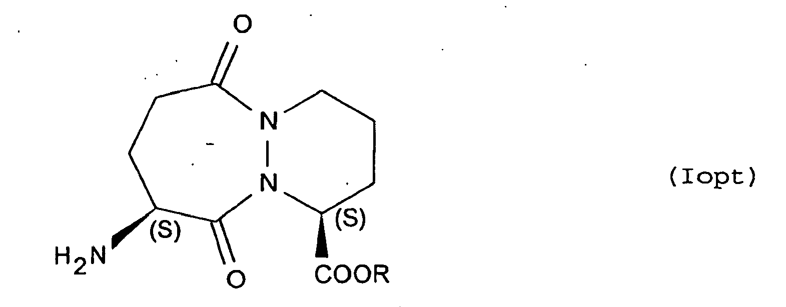 sous forme SS, dans laquelle la fonction amine est libre ou protégée et R conserve sa signification précédente.

L'invention a plus spécialement pour objet l'application des composés de formule (IA) définis ci-dessus, à la préparation des composés de formule (IAopt) : 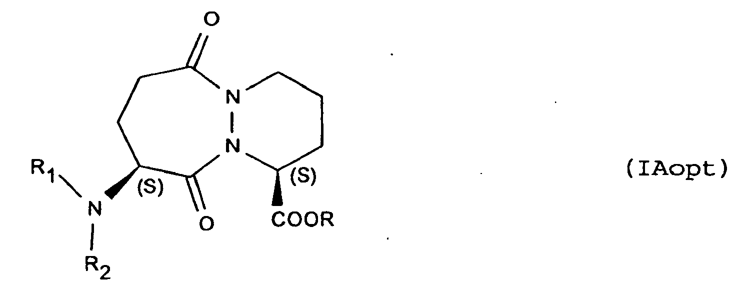 sous forme SS, dans laquelle R, R₁ et R₂ conservent leur signification précédente.

L'invention a plus particulièrement pour objet l'application caractérisée en ce que R représente un radical méthyle, et celle dans laquelle la fonction amine est protégée sous forme de phtalimido.

L'invention a plus particulièrement pour objet l'application caractérisée en ce que l'agent de déracémisation est une base, plus spécialement une base forte, par exemple un alcoolate alcalin ou alcalinoterreux comme le méthylate de sodium ou de potassium, le terbutylate de sodium ou de potassium, ou une amine lithiée comme la LDA.

L'invention a tout particulièrement pour objet l'application décrite ci-après dans la partie expérimentale pour préparer le :
- (1S-cis)-9-(1,3-dihydro-1,3-dioxo-2H-isoindol-2-yl)-3,4,7,8,9,10-hexahydro-6,10-dioxo-6H-pyridazino[1,2-a][1,2] diazépine-1-carboxylate de méthyle.

Le produit de formule (I) de configuration SS dans lequel R est un radical terbutyle et l'amine est protégée sous forme de phtalimido, est décrit par exemple dans le brevet EP 94095, c'est un produit intermédiaire dans la synthèse de produits présentant des propriétés thérapeutiques.

Les produits de formule (I) peuvent être d'une façon générale utilisés pour la synthèse de médicaments comme il est indiqué dans le brevet ci-dessus.

L'exemple suivant illustre l'invention sans la limiter.

### EXEMPLE : (1S-cis)-9-(1,3-dihydro-1,3-dioxo-2H-isoindol-2-yl)octahydro-6,10-dioxo-6H-pyridazino[1,2-a][1,2]diazépine-1-carboxylate de méthyle et (1R-trans)-9-(1,3-dihydro-1,3-dioxo-2H-isoindol-2-yl) octahydro-6,10-dioxo-6H-pyridazino[1,2-a][1,2]diazépine-1-carboxylate de méthyle.

### a) Préparation d'acide 2,5-dibromopentanoïque

On ajoute 39 ml de brome dans un mélange de 106 g d'acide 5-bromo pentanoïque et de 1 ml de tribromure de phosphore. On porte le mélange réactionnel à 70~80°C pendant 16 h 30. On amène le milieu réactionnel à 100°C pendant 15 minutes, on laisse revenir à la température ambiante. On obtient 147 g de produit recherché.

### b) Préparation du 2,5-dibromopentanoate d'éthyle

On ajoute 24,37 g de chlorure d'oxalyle dans un mélange renfermant 50 g du produit préparé au stade précédent, 15 gouttes de DMF, et 300 ml de chlorure de méthylène. On maintient le mélange réactionnel sous agitation, à la température ambiante, jusqu'à ce que la réaction soit complète. On refroidit le mélange réactionnel à 10°C et ajoute 50 ml d'alcool éthylique. On agite pendant 30 minutes à 10°C, laisse revenir à la température ambiante et agite pendant 3 heures à la température ambiante. On amène à sec et obtient le produit recherché.

### c) Préparation de 1,2-hydrazinedicarboxylate de bis (phénylméthyl)

On place sous azote 1,5 litre de méthanol et 25 g d'hydrazine monohydrate à 80 %. On refroidit à 0°C et introduit, à 0°C, 75 g de chloroformiate de benzyle puis de nouveau 75 g de chloroformiate de benzyle en même temps qu'une solution de 93 g de carbonate de sodium dans 1100 ml d'eau déminéralisée. On maintient le mélange réactionnel pendant 1 heure à 0°C, essore et lave par déplacement avec un mélange de 100 ml de méthanol et 100 ml d'eau, puis on lave par déplacement avec 500 ml d'eau à 0°C. On sèche et obtient 107,6 g de produit recherché.

### d) Préparation de (S)-tétrahydro-1,2,3-pyridazinetricarboxylate de 3-éthyle-1,2-bis(phénylméthyle) et (R)-tétrahydro-1,2,3-pyridazinetricarboxylate de 3-éthyle-1,2-bis (phénylméthyle)

On introduit à 20-25°C, une suspension de 12,1 g du produit du 2,5-dibromopentanoate d'éthyle et 50 cm³ de diglyme dans une suspension renfermant 10,42 g de 1,2-hydrazinedicarboxylate de bis(phénylméthyle), 65 ml de diglyme et 8,26 g de carbonate de potassium.

On chauffe à 90°C la suspension obtenue. On maintient l'agitation pendant 48 heures. On refroidit à 20°C, verse dans une solution renfermant 50 ml d'acide chlorhydrique 2N et 150 ml d'un mélange d'eau et glace. On extrait à l'acétate d'éthyle, lave à l'eau, sèche. On filtre, rince à l'acétate d'éthyle et sèche. On chromatographie sur silice (élution heptane 40, AcOEt 20) le produit obtenu et obtient 10,71 g de produit recherché.

### e) Préparation de (S)-tétrahydro-1,3(2H)-pyridazinedicarboxylate de 1-(phénylméthyle) et (R)-tétrahydro-1,3(2H)-pyridazinedicarboxylate de 1-(phénylméthyle)

On introduit une solution renfermant 23,25 g du produit du stade précédent et 80 ml d'éthanol dans 338 ml d'une solution d'hydroxyde de sodium dans l'éthanol à 40 g par litre. On maintient l'agitation pendant 5 heures 30 minutes et ajoute 57 ml de soude 2N. On maintient le mélange réactionnel sous agitation pendant 30 heures. On ajoute 141 ml d'une solution d'acide chlorhydrique 2N. On distille 260 ml de mélange réactionnel sous 80-90 millibars. On extrait au dichlorométhane, ajoute 20 ml d'éthanol, lave avec un mélange eau-solution normale de soude. On extrait les phases aqueuses au dichlorométhane. On réunit les phases aqueuses, agite et acidifie avec 135 ml d'une solution d'acide chlorhydrique 2N. On extrait au dichlorométhane, lave à l'eau, sèche, filtre et lave au chlorure de méthylène. On concentre et sèche. On ajoute 146 ml d'éther isopropylique, agite 1 heure à 20°C, filtre, lave, concentre et sèche. On obtient 11,41 g de produit recherché.

### f) Préparation de (S)-tétrahydro-1,3(2H)-pyridazinedicarboxylate de 3-méthyle 1-(phénylméthyle) et (R)-tétrahydro-1,3(2H)-pyridazinedicarboxylate de 3-méthyle 1-(phénylméthyle)

On ajoute 220 ml de méthanol et l'acide paratoluène sulfonique déshydraté (préparé à partir d'APTS monohydraté et 12 ml de dichlorométhane) à 11,05 g du produit préparé au stade précédent. On maintient la suspension obtenue sous agitation pendant 15 heures. On chauffe à 65°C et maintient l'agitation pendant 6 heures 30 minutes. On refroidit à 5°C, ajoute 5,5 ml d'une solution de bicarbonate de soude à 10 %. On concentre sous pression réduite, reprend avec un mélange de 100 ml de dichlorométhane et 100 ml d'eau. On agite, décante, lave la phase organique, extrait au dichlorométhane, sèche, filtre et concentre. On obtient 11,39 g de produit recherché.

### g) Préparation de [3S-[2(R*),3R*]]-2-[2-(1,3-dihydro-1,3-dioxo-2H-isoindol-2-yl)-1,5-dioxo-5-(phénylméthoxy)pentyl] tétrahydro-1,3(2H)-pyridazinedicarboxylate de 3-méthyle 1-(phénylméthyle) et [3R-[2(S*),3R*]]-2-[2-(1,3-dihydro-1,3-dioxo-2H-isoindol-2-yl)-1,5-dioxo-5-(phénylméthoxy)pentyl]tétrahydro-1,3(2H)-pyridazinedicarboxylate de 3-méthyle 1-(phénylméthyle)

On introduit en 1 heure vers 4°C, une solution renfermant 11,01 g du produit préparé au stade précédent et 50 ml de dichlorométhane dans une solution renfermant 19,88 g de (S)-gamma-(chlorocarbonyl)-1,3-dihydro-1,3-dioxo-2H-isoindole-2-butanoate de phénylméthyle et 100 ml de dichlorométhane. On agite pendant une demi-heure à 4°C et introduit en 1 heure et demie 4,15 ml de pyridine dans 25 ml de dichlorométhane. On maintient l'agitation pendant 15 heures en laissant revenir lentement à la température ambiante. On concentre, sous pression réduite, reprend avec 200 ml d'acétate d'étyle, lave avec une solution saturée de carbonate acide de sodium, agite une demi-heure, décante, lave avec une solution saturée de carbonate acide de sodium, agite et décante. On lave avec une solution renfermant 5 ml d'une solution normale d'acide chlorhydrique et 25 ml d'eau, puis avec une solution aqueuse saturée de chlorure de sodium et sèche. On extrait à l'acétate d'éthyle, concentre et sèche. On obtient 25,2 g de produit recherché.

### h) Préparation d'acide [6S-[1(R*),6R*]]-1,3-dihydro-1,3-dioxo-gamma-[[6-(méthoxycarbonyl)-tétrahydro-1(2H)-pyridazinyl]carbonyl]-2H-isoindole-2-butanoïque et acide [6R-[1(S*),6R*]]-1,3-dihydro-1,3-dioxo-gamma-[[6-(méthoxycarbonyl)-tétrahydro-1(2H)-pyridazinyl]carbonyl]-2H-isoindole-2-butanoïque

On introduit dans un appareil à hydrogène 20,23 g de produit du stade précédent, 250 ml de THF et 3,03 g de palladium à 10 % sur charbon. On fait passer l'hydrogène pendant 3 heures, ajoute à nouveau 3,03 g de catalyseur. On poursuit l'hydrogénation pendant 22 heures. On filtre, lave avec du THF et évapore. On ajoute 25 ml d'isopropanol, concentre, chasse le THF, ajoute 15 ml d'isopropanol. On obtient une suspension à laquelle on ajoute 100 ml d'éther isopropylique. On agite sous azote pendant 2 heures, essore, lave à l'éther isopropylique à 5 % d'isopropanol. On essore, sèche et obtient 9,5 g de produit recherché.

### i) Préparation de (1S-cis)-9-(1,3-dihydro-1,3-dioxo-2H-isoindol-2-yl)octahydro-6,10-dioxo-6H-pyridazino[1,2-a] [1,2]diazépine-1-carboxylate de méthyle et (1R-trans)-9-(1,3-dihydro-1,3-dioxo-2H-isoindol-2-yl) octahydro-6,10-dioxo-6H-pyridazino[1,2-a][1,2]diazéipine-1-carboxylate de méthyle.

On ajoute à 5 °C une solution renfermant 1 ml de chlorure de thionyle et 40 ml de chlorure de méthylène dans un mélange renfermant 4,038 g de produit du stade précédent, 40 ml de dichlorométhane et 0,4 ml de diméthylformamide. On agite pendant 3 heures et demie. On laisse la température remonter vers 20°C, agite une heure et demie. On concentre. On ajoute une solution renfermant 0,15 ml de chlorure de thionyle et 5 ml de chlorure de méthylène. On maintient le mélange réactionnel sous agitation vers 20°C pendant 16 heures. On refroidit vers 5°C et introduit 27 ml d'une solution aqueuse saturée en carbonate acide de sodium. On agite une demi-heure. On décante et lave avec une solution renfermant 10 ml de bicarbonate de soude et 40 ml d'eau déminéralisée. On agite 3 minutes, décante, extrait les phases aqueuses au chlorure de méthylène, sèche, filtre, lave au chlorure de méthylène et concentre sous pression réduite. On obtient 3,85 g de produit recherché.

### Application (1S-cis)-9-(1,3-dihydro-1,3-dioxo-2H-isoindol-2yl)octahydro-6,10-dioxo-6H-pyridazino[1,2-a][1,2]diazépine-1-carboxylate de méthyle

On introduit à une température de -45°/-48°C en 1 heure 30 minutes, une solution renfermant 0,029 g de terbutylate de potassium et 0,3 ml de DMF dans un mélange renfermant 0,194 g du produit de l'exemple 1, 1,5 ml de diméthylformamide et 0,75 ml de terbutanol. On maintient le mélange sous agitation pendant 1 heure et introduit après refroidissement à -50°C, 0,4 g de chlorure d'ammonium en poudre. On agite 10 minutes à -45°C, ajoute successivement 2 fois 1 ml de chlorure d'ammonium à 20% en agitant à nouveau 10 minutes après chaque addition. On ajoute 2 ml d'eau déminéralisée. On extrait à l'acétate d'éthyle, lave à l'eau déminéralisée, décante, concentre et sèche. On obtient 0,166 g de produit. α_{D} = -75,3° (1 % dans méthanol).

## Revendications

1. Les composés de formule (I) : 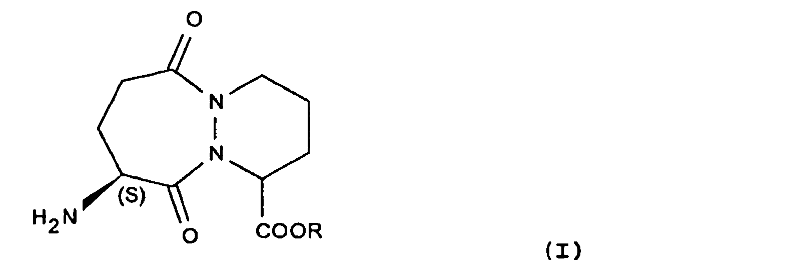 sous forme de mélange 9S1R + 9S1S, dans laquelle R représente un atome d'hydrogène, un radical alkyle ou aralkyle renfermant jusqu'à 18 atomes de carbone, la fonction amine pouvant être libre ou protégée.

2. Les composés de formule (I) définis à la revendication 1, répondant à la formule (IA) : 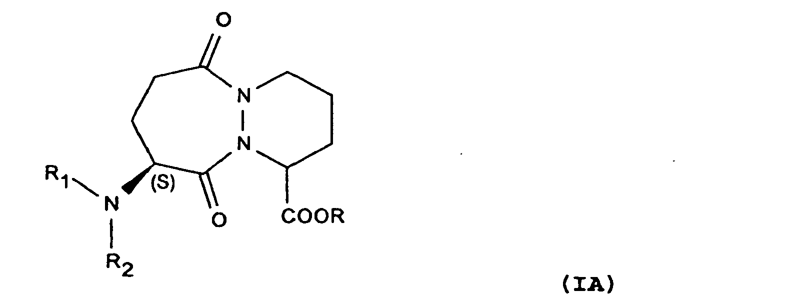 sous forme de mélange 9S1R + 9S1S, dans laquelle R est tel que défini à la revendication 1 et/ou bien R₁ représente un radical : 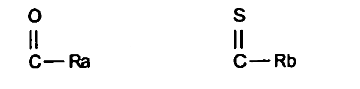 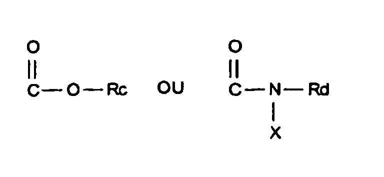 Ra, Rb, Rc et Rd représentant un radical alkyle ou aryle renfermant jusqu'à 18 atomes de carbone, ou un radical mono-, bi- ou tricyclique renfermant un ou plusieurs hétéroatomes,
X représentant un atome d'hydrogène, un radical alkyle renfermant jusqu'à 8 atomes de carbone ou un radical aryle renfermant jusqu'à 14 atomes de carbone,
et R₂ représente un atome d'hydrogène,
ou bien R₁ et R₂ forment ensemble un radical mono-, bi- ou tricyclique renfermant un ou plusieurs hétéroatomes.

3. Les composés de formule (IA) définis à la revendication 1 ou 2 dans lesquels R₁ et R₂ forment ensemble un radical polycyclique renfermant un ou plusieurs hétéroatomes sous forme de mélange 9S1R + 9S1S.

4. Les composés de formule (IA) définis à la revendication 3, répondant à la formule (IA₁) : 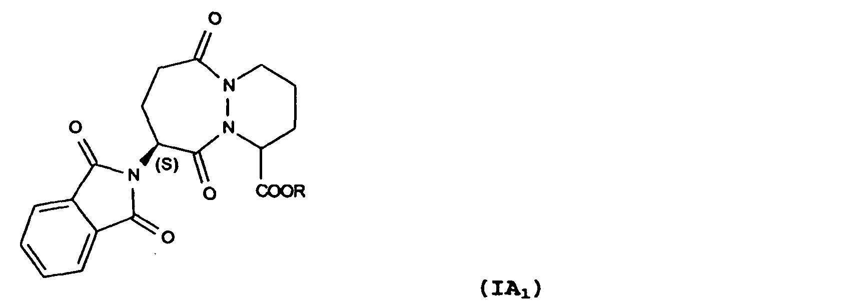 sous forme de mélange 9S1R + 9S1S.

5. Les composés de formule (I) définis à l'une quelconque des revendications 1 à 4, dans laquelle R représente un radical méthyle.

6. Le mélange racémique des composés de formule (I) définis à la revendication 1 dont les noms suivent :
(1S-cis)-9-(1,3-dihydro-1,3-dioxo-2H-isoindol-2-yl)octahydro-6,10-dioxo-6H-pyridazino[1,2-a][1,2]diazépine-1-carboxylate de méthyle et (1R-trans)-9-(1,3-dihydro-1,3-dioxo-2H-isoindol-2-yl)
octahydro-6,10-dioxo-6H-pyridazino[1,2-a][1,2]diazépine-1-carboxylate de méthyle.

7. Procédé de préparation des composés de formule (I) définis à l'une quelconque des revendications précédentes, **caractérisé en ce que** l'on soumet un composé de formule (II) : 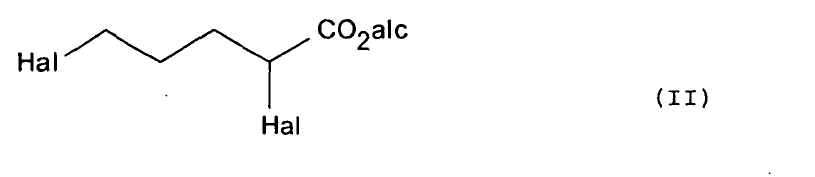 dans laquelle alc représente un radical alkyle renfermant jusqu'à 8 atomes de carbone et Hal représente un atome d'halogène, à l'action d'un composé de formule (III) : 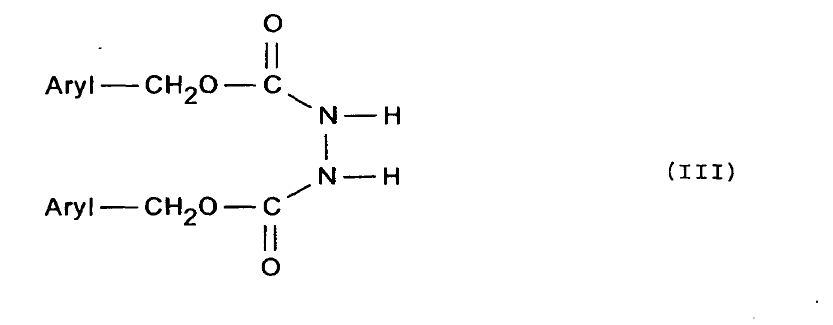 dans laquelle Aryl représente un radical aryle renfermant jusqu'à 14 atomes de carbone, pour obtenir le composé de formule (IV) : 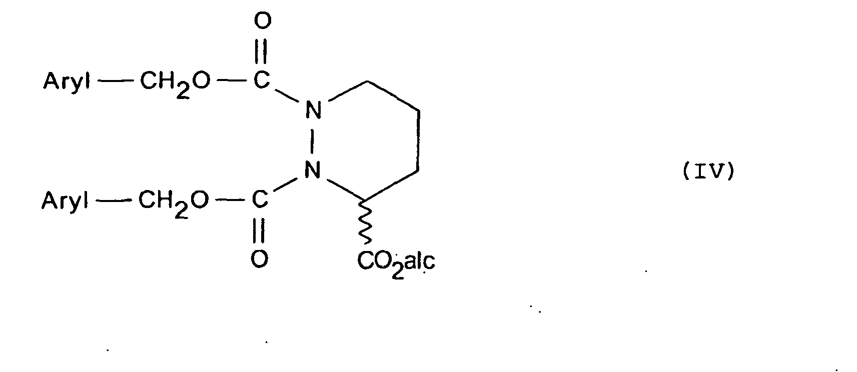 que l'on soumet à l'action d'un agent basique, pour obtenir le composé de formule (V) : 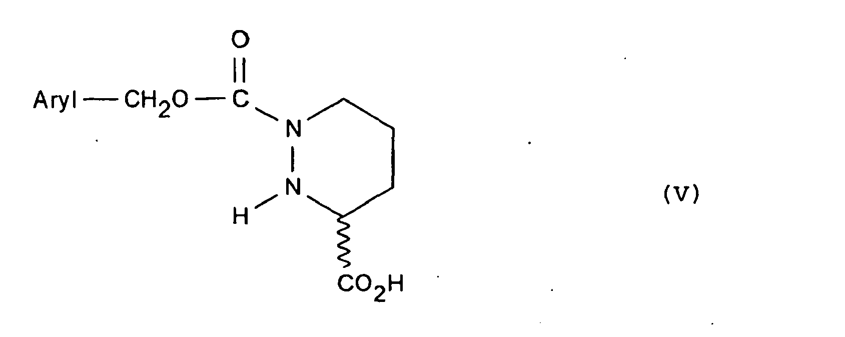 que l'on soumet éventuellement à l'action d'un agent d'alkylation pour obtenir le composé de formule (VI) : 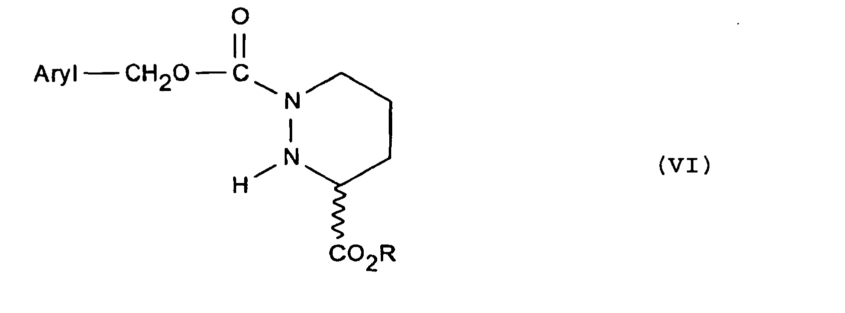 que l'on soumet à l'action d'un composé de formule (VII) : 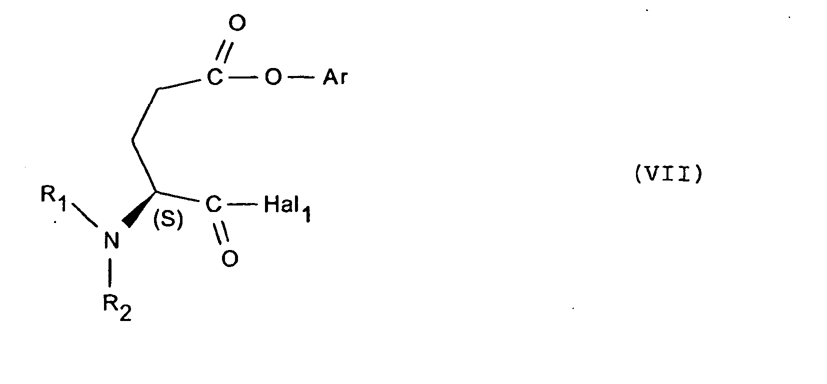 dans laquelle Hall représente un atome d'halogène et Ar représente un radical aryle ou aralkyle renfermant jusqu'à 18 atomes de carbone, R₁ et R₂ conservant la même définition que dans la revendication 2, pour obtenir le composé de formule (VIII) : 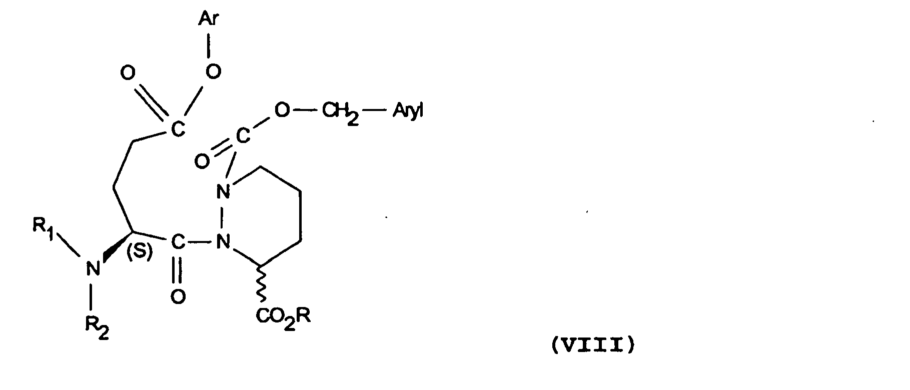 sous forme de mélange 9S1R + 9S1S, que l'on soumet à l'action d'un agent d'hydrogénation pour obtenir le composé de formule (IX) : 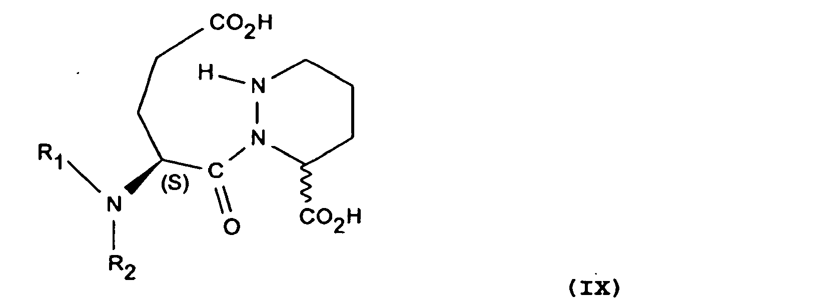 sous forme de mélange 9S1R + 9S1S, que l'on soumet à l'action d'un agent de condensation pour obtenir le composé de formule (IA) correspondant, puis si désiré, libère la fonction amine pour obtenir le composé de formule (I) dans lequel la fonction amine est libre.

8. A titre de produits chimiques, les composés de formules (IV), (VIII) et (IX) définis à la revendication 7.

9. Application des composés de formule (I) définis à l'une quelconque des revendications 1 à 6, sous forme de mélanges 9S1S,9S1R, **caractérisée en ce que** l'on soumet un composé de formule (I) à l'action d'un agent de déracémisation du carbone asymétrique porté par le cycle en position 1, à la préparation du composé de formule (Iopt) : 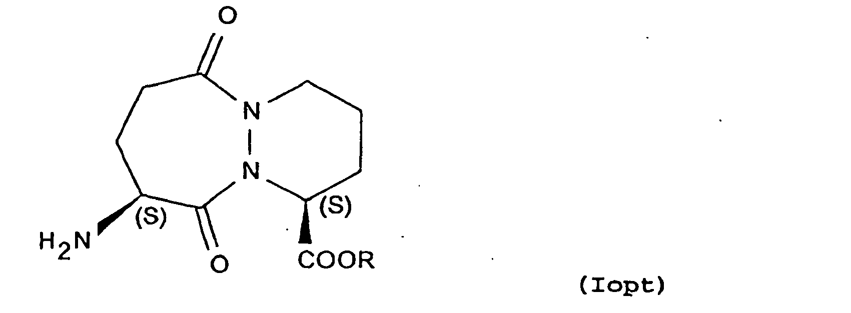 sous forme 9S1S, dans laquelle la fonction amine est libre ou protégée et R est tel que défini à la revendication 1.

10. Application de la revendication 9 des composés de formule (IA) définis à l'une quelconque des revendications 2 à 6, à la préparation des composés de formule (IAopt) : 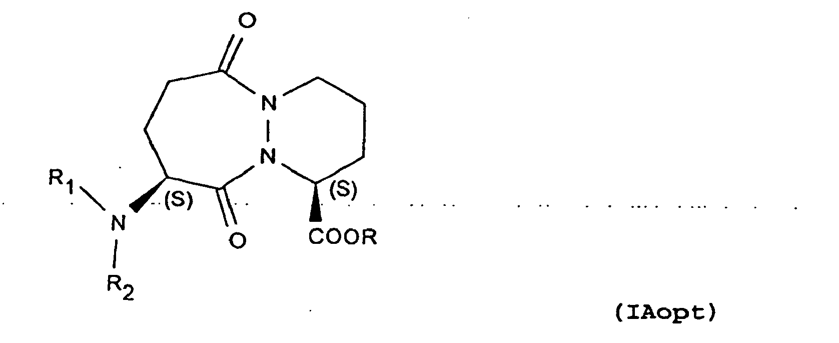 sous forme 9S1S, dans laquelle R, R₁ et R₂ conservent la même définition qu'à la revendication 2.

11. Application selon la revendication 9 ou 10, **caractérisée en ce que** R représente un radical méthyle.

12. Application selon la revendication 9, 10 ou 11, **caractérisée en ce que** la fonction amine est protégée sous forme de phtalimido.

13. Application selon l'une quelconque des revendications 9 à 12, **caractérisée en ce que** l'agent de déracémisation est une base.

14. Application selon la revendication 13, **caractérisée en ce que** la base forte est un alcoolate alcalin ou alcalino terreux ou une amine lithiée.

15. Application selon l'une quelconque des revendications 9 à 14, **caractérisée en ce que** le produit de départ est le mélange racémique selon la revendication 6 et le produit préparé est le :
- (1S-cis)-9-(1,3-dihydro-1,3-dioxo-2H-isoindol-2-yl)-octahydro-6,10-dioxo-6H-pyridazino[1,2-a][1,2] diazépine-1-carboxylate de méthyle.

## Claims

1. The compounds of formula (I): 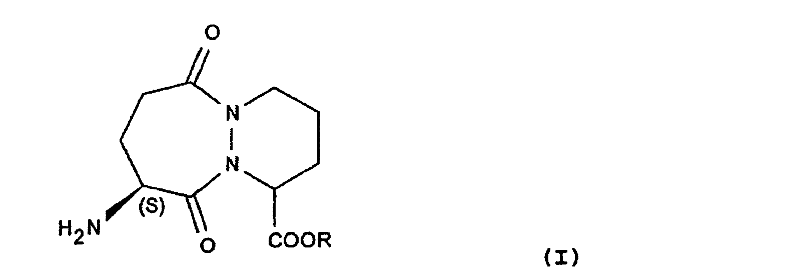 in the form of a (9S,1R) + (9S,1S) mixture, in which formula R represents a hydrogen atom or an alkyl or aralkyl radical comprising up to 18 carbon atoms, it being possible for the amine functional group to be free or protected.

2. The compounds of formula (I) defined in Claim 1, corresponding to the formula (IA): 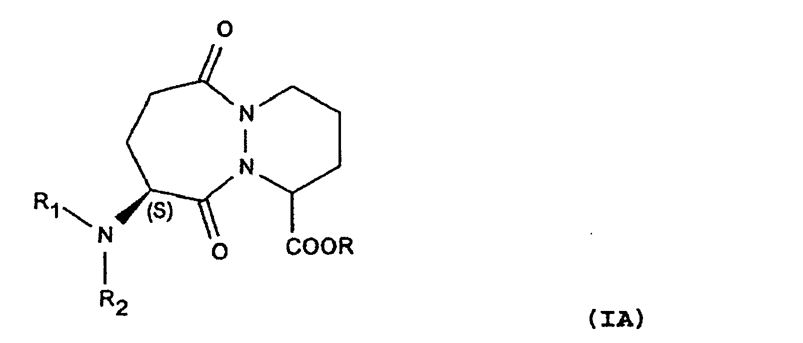 in the form of an (9S,1R) + (9S,1S) mixture, in which formula R is as defined in Claim 1 and/or either R₁ represents a radical: 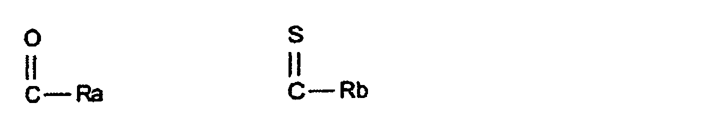 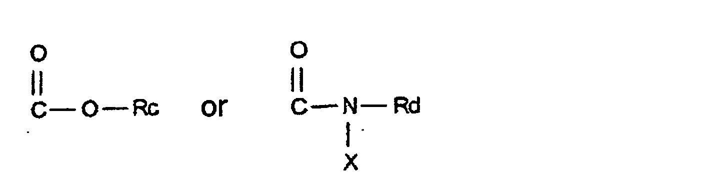 Ra, Rb, Rc and Rd representing an alkyl or aryl radical comprising up to 18 carbon atoms or a mono-, bi- or tricyclic radical comprising one or more heteroatoms,
X representing a hydrogen atom, an alkyl radical comprising up to 8 carbon atoms or an aryl radical comprising up to 14 carbon atoms,
and R₂ represents a hydrogen atom,
or else R₁ and R₂ together form a mono-, bi- or tricyclic radical comprising one or more heteroatoms.

3. The compounds of formula (IA) defined in Claim 1 or 2, in which R₁ and R₂ together form a polycyclic radical comprising one or more heteroatoms in the form of a (9S,1R) + (9S,1S) mixture.

4. The compounds of formula (IA) defined in Claim 3, corresponding to the formula (IA₁): 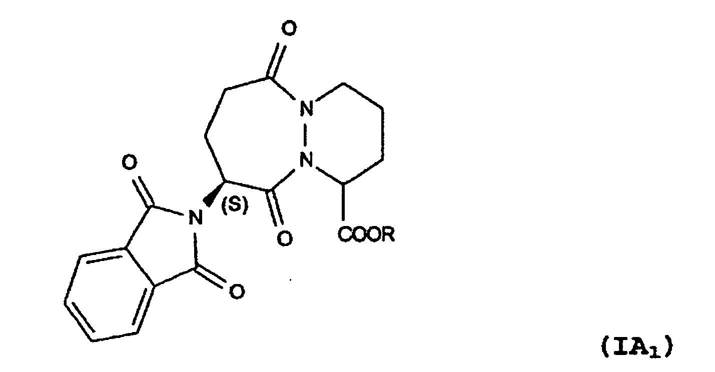 in the form of an (9S,1R) + (9S,1S) mixture.

5. The compounds of formula (I) defined in any one of Claims 1 to 4, in which R represents a methyl radical.

6. The racemic mixture of the compounds of formula (I) defined in Claim 1, the names of which follow: methyl (1S-cis)-9-(1,3-dihydro-1,3-dioxo-2H-isoindol-2-yl)octahydro-6,10-dioxo-6H-pyridazino[1,2-a][1,2]-diazepine-1-carboxylate and methyl (1R-trans)-9-(1,3-dihydro-1,3-dioxo-2H-isoindol-2-yl)octahydro-6,10-dioxo-6H-pyridazino[1,2-a][1,2]diazepine-1-carboxylate.

7. Process for the preparation of the compounds of formula (I) defined in any one of the preceding claims, **characterized in that** a compound of formula (II): 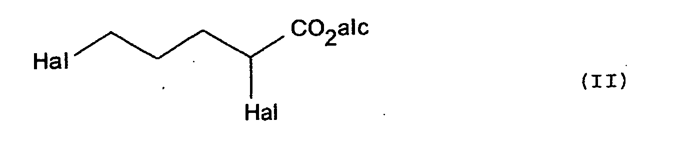 in which alc represents an alkyl radical comprising up to 8 carbon atoms and Hal represents a halogen atom, is subjected to the action of a compound of formula (III): 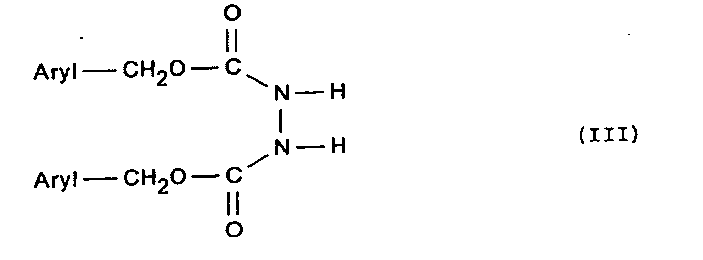 in which Aryl represents an aryl radical comprising up to 14 carbon atoms, in order to obtain the compound of formula (IV): 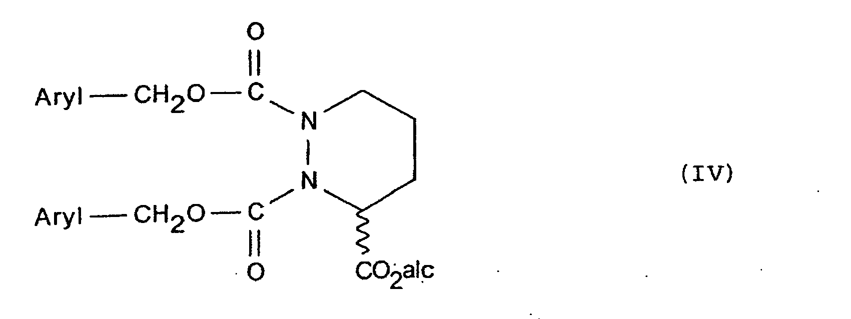 which is subjected to the action of a basic agent, in order to obtain the compound of formula (V): 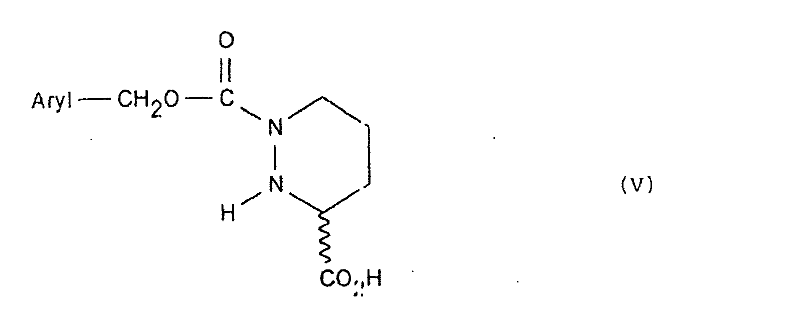 which is optionally subjected to the action of an alkylating agent, in order to obtain the compound of formula (VI): 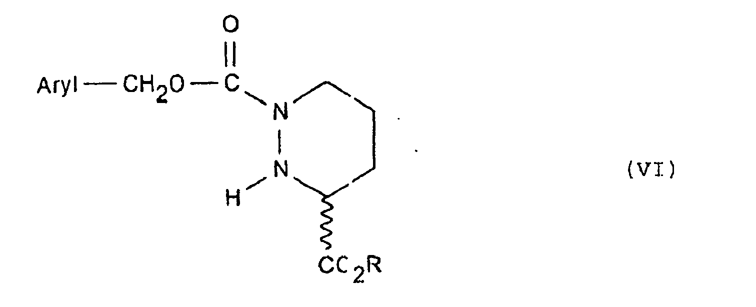 which is subjected to the action of a compound of formula (VII): 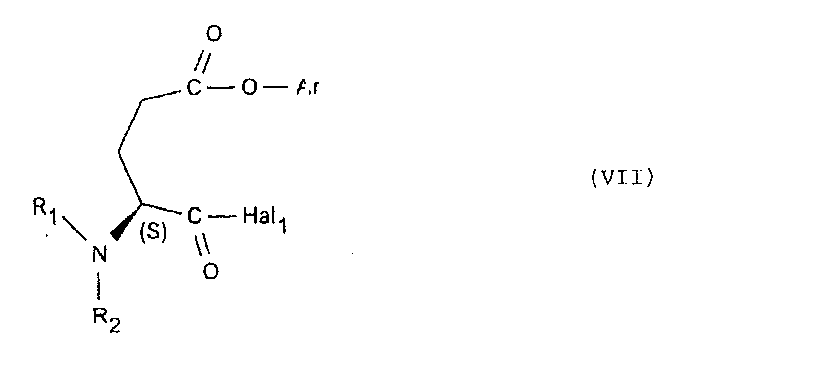 in which Hal₁ represents a halogen atom and Ar represents an aryl or aralkyl radical comprising up to 18 carbon atoms, R₁ and R₂ retaining the same definitions as in Claim 2, in order to obtain the compound of formula (VIII): 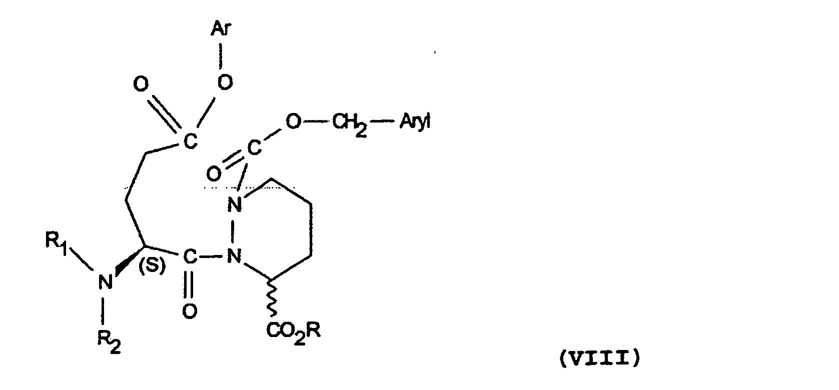 in the form of an (9S,1R) + (9S,1S) mixture, which is subjected to the action of a hydrogenating agent, in order to obtain the compound of formula (IX): 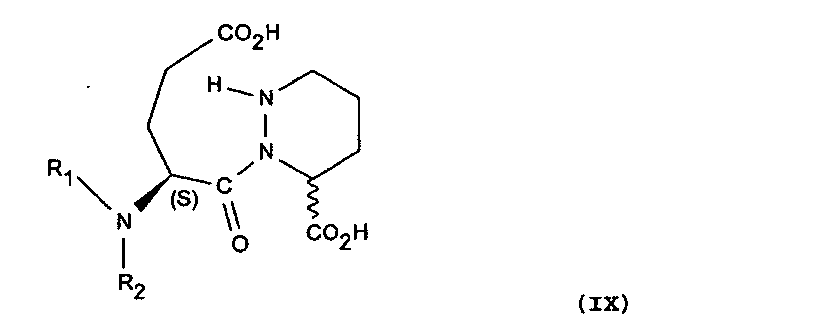 in the form of a (9S,1R) + (9S,1S) mixture, which is subjected to the action of a coupling agent, in order to obtain the corresponding compound of formula (IA), and then, if desired, the amine functional group is released, in order to obtain the compound of formula (I) in which the amine functional group is free.

8. As chemical products, the compounds of formulae (IV), (VIII) and (IX) defined in Claim 7.

9. Application of the compounds of formula (I) defined in any one of Claims 1 to 6 in the form of (9S,1S) + (9S,1R) mixtures, **characterized in that** a compound of formula (I) is subjected to the action of an agent for deracemizing the asymmetric carbon carried by the ring in the 1-position, in the preparation of the compound of formula (Iopt): 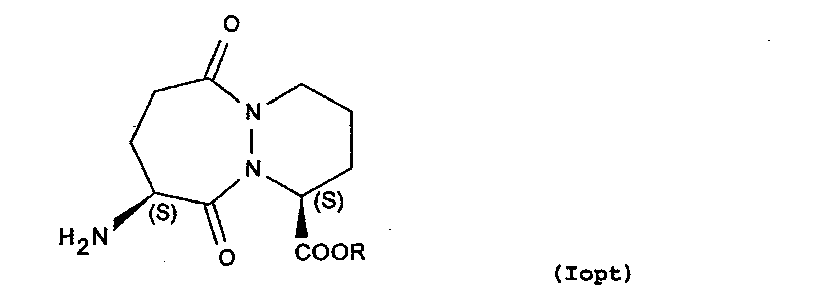 in the (9S,1S) form, in which formula the amine functional group is free or protected and R is as defined in Claim 1.

10. Application according to claim 9 of the compounds of formula (IA) defined in any one of Claims 2 to 6, in the preparation of the compounds of formula (IAopt): 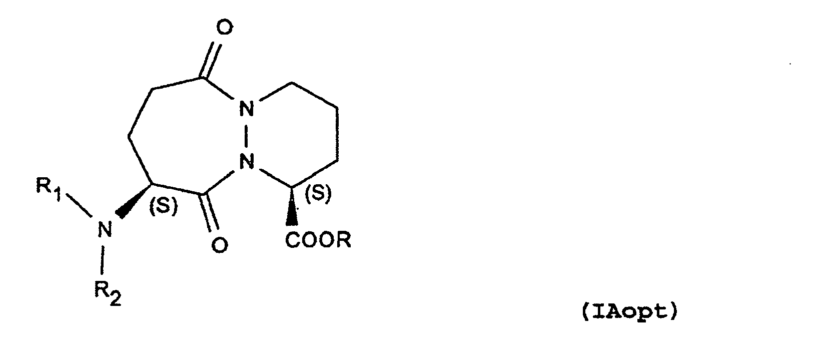 in the (9S,1S) form, in which formula R, R₁ and R₂ retain the same definitions as in Claim 2.

11. Application according to Claim 9 or 10, **characterized in that** R represents a methyl radical.

12. Application according to Claim 9, 10 or 11, **characterized in that** the amine functional group is protected in the phthalimido form.

13. Application according to any one of Claims 9 to 12, **characterized in that** the deracemizing agent is a base.

14. Application according to Claim 13, **characterized in that** the strong base is an alkali metal or alkaline earth metal alkoxide or a lithium amide.

15. Application according to any one of Claims 9 to 14, **characterized in that** the starting material is the racemic mixture according to Claim 6 and the product prepared is:
- methyl (1S-cis)-9-(1,3-dihydro-1,3-dioxo-2H-isoindol-2-yl)-octahydro-6,10-dioxo-6H-pyridazino[1,2-a][1,2]diazepine-1-carboxylate.

## Patentansprüche

1. Verbindungen der Formel (I) 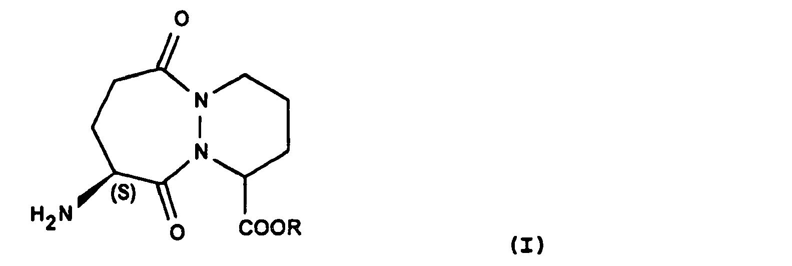 in Form der Mischung 9S1R + 9S1S, in der R ein Wasserstoffatom, einen Rest Alkyl oder Aralkyl, umfassend bis zu 18 Kohlenstoffatome, darstellt, wobei die Aminfunktion frei oder geschützt sein kann.

2. Verbindungen der Formel (I) wie in Anspruch 1 definiert, entsprechend der Formel (IA): 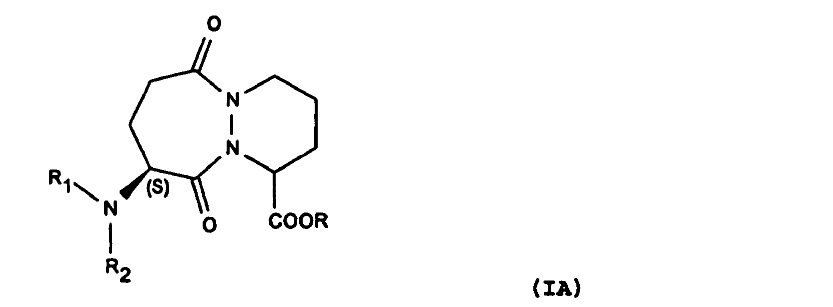 in Form der Mischung 9S1R + 9S1S, in der R wie in Anspruch 1 definiert ist, und/oder R₁ einen Rest 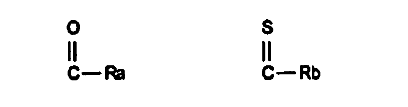 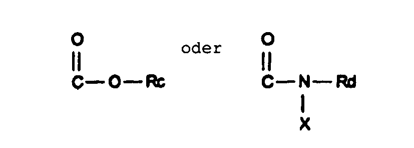 darstellt, Ra, Rb, Rc und Rd einen Rest Alkyl oder Aryl, umfassend bis zu 18 Kohlenstoffatome, oder einen mono-, bi- oder tricyclischen Rest darstellen, umfassend ein oder mehrere Heteroatome,
X ein Wasserstoffatom, einen Rest Alkyl, umfassend bis zu 8 Kohlenstoffatome oder einen Rest Aryl, umfassend bis zu 14 Kohlenstoffatome, bedeutet, und
R₂ ein Wasserstoffatom darstellt,
oder auch R₁ und R₂ zusammen einen mono-, bi- oder tricyclischen Rest bilden, umfassend ein oder mehrere Heteroatome.

3. Verbindungen der Formel (IA) wie in Anspruch 1 oder 2 definiert, in der R₁ und R₂ zusammen einen polycyclischen Rest bilden, umfassend ein oder mehrere Heteroatome, in Form der Mischung 9S1R + 9S1S.

4. Verbindungen der Formel (IA) wie in Anspruch 3 definiert, entsprechend der Formel (IA₁) 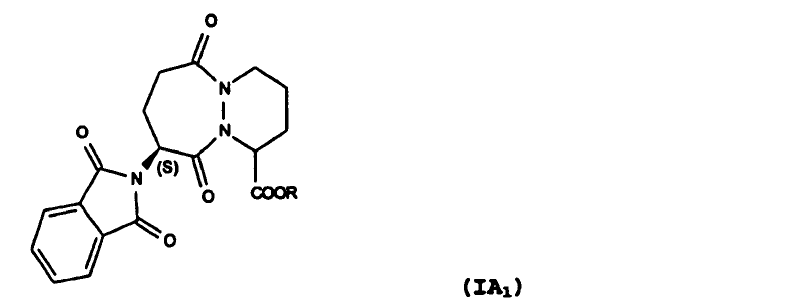 in Form der Mischung 9S1R + 9S1S.

5. Verbindungen der Formel (I) wie in irgendeinem der Ansprüche 1 bis 4 definiert, in der R einen Rest Methyl darstellt.

6. Racemische Mischung der Verbindungen der Formel (I) wie in Anspruch 1 definiert, deren Namen folgen:
(1S-cis)-9-(1,3-Dihydro-1,3-dioxo-2H-isoindol-2-yl)-octahydro-6,10-dioxo-6H-pyridazino[1,2-a]-[1,2]diazepin-1-carbonsäure-methylester und
(1R-trans)-9-(1,3-Dihydro-1,3-dioxo-2H-isoindol-2-yl)-octahydro-6,10-dioxo-6H-pyridazino[1,2-a]-[1,2]diazepin-1-carbonsäure-methylester.

7. Verfahren zur Herstellung der Verbindungen der Formel (I) wie in irgendeinem der vorstehenden Ansprüche definiert, **dadurch gekennzeichnet, daß** man eine Verbindung der Formel (II) 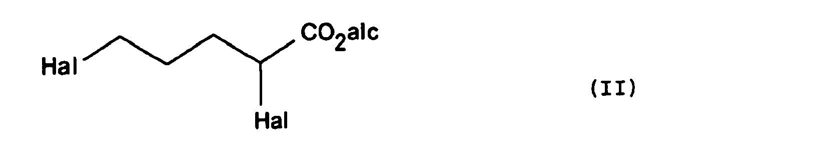 in der alc einen Rest Alkyl darstellt, umfassend bis zu 8 Kohlenstoffatome, und Hal ein Halogenatom bedeutet, der Einwirkung einer Verbindung der Formel (III) 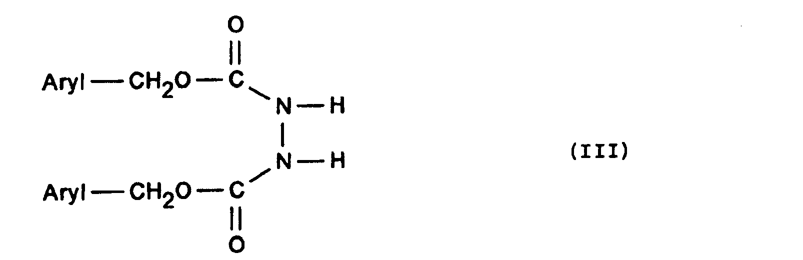 unterzieht, in der Aryl einen Rest Aryl darstellt, umfassend bis zu 14 Kohlenstoffatome, um die Verbindung der Formel (IV) 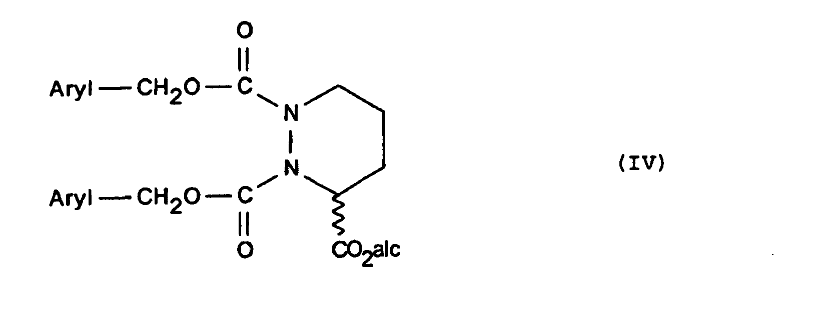 zu erhalten, die man der Einwirkung eines basischen Mittels unterzieht, um die Verbindung der Formel (V) 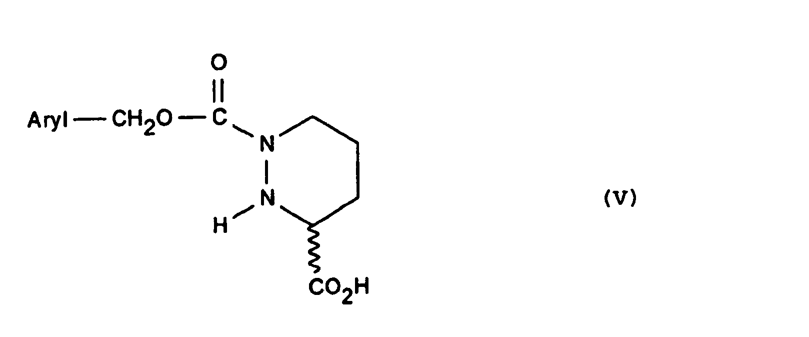 zu erhalten, die man gegebenenfalls der Einwirkung eines Alkylierungsmittel unterzieht, um die Verbindung der Formel (VI) 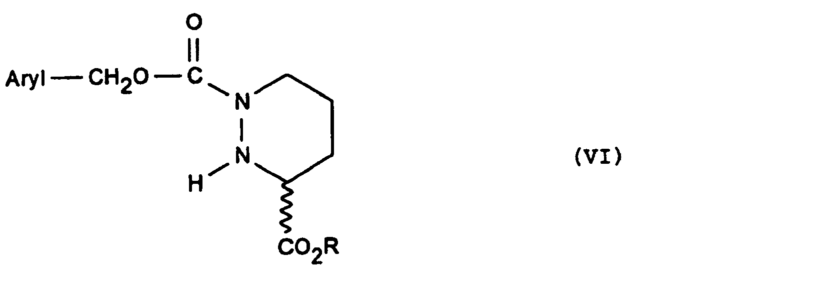 zu erhalten, die man der Einwirkung einer Verbindung der Formel (VII) 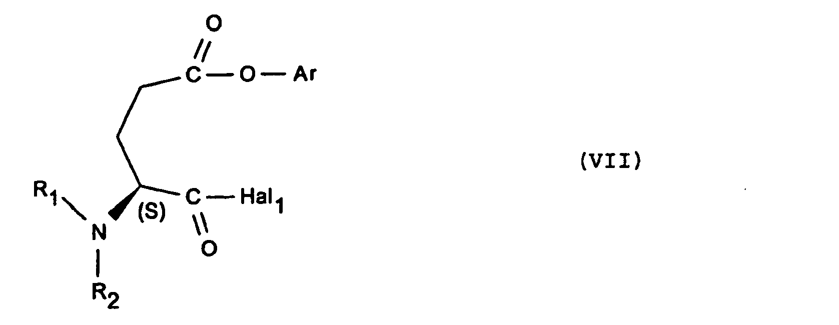 unterzieht, in der Hall ein Halogenatom darstellt und Ar einen Rest Aryl oder Aralkyl bedeutet, umfassend bis zu 18 Kohlenstoffatome, R₁ und R₂ die gleiche Definition wie in Anspruch 2 beibehalten, um die Verbindung der Formel (VIII) 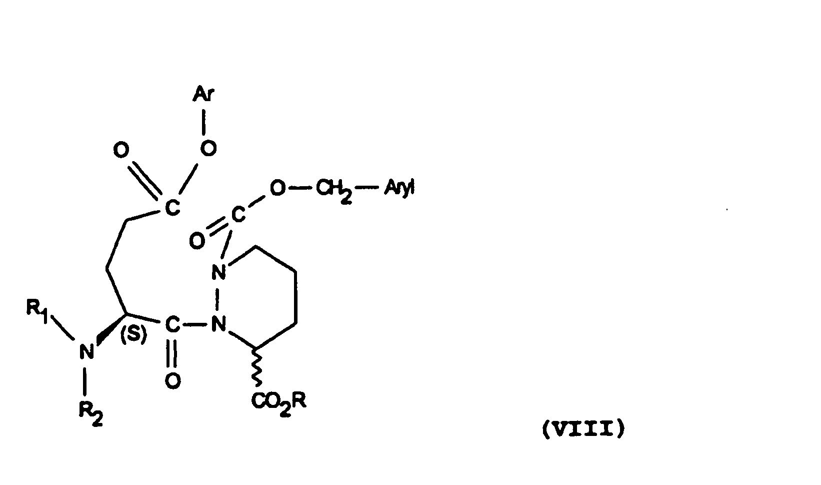 in Form der Mischung 9S1R + 9S1S zu erhalten, die man der Einwirkung eines Hydrierungsmittels unterzieht, um die Verbindung der Formel (IX) 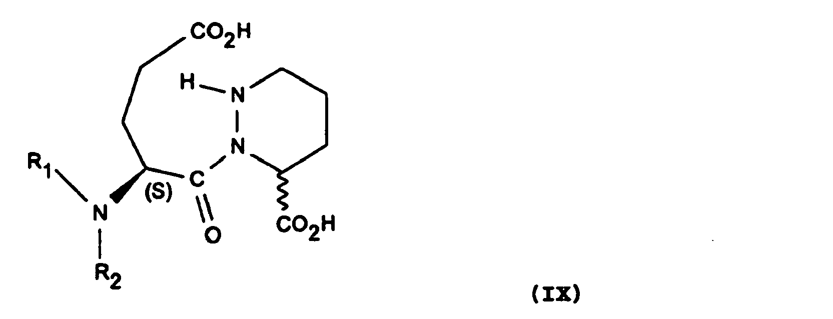 in Form der Mischung 9S1R + 9S1S zu erhalten, die man der Einwirkung eines Kondensationsmittels unterzieht, um die entsprechende Verbindung der Formel (IA) zu erhalten, bei der man dann, wenn erwünscht, die Aminfunktion freisetzt, um die Verbindung der Formel (I) zu erhalten, bei der die Aminfunktion frei ist.

8. Als chemische Produkte die Verbindungen der Formel (IV), (VIII) und (IX) wie in Anspruch 7 definiert.

9. Verwendung der Verbindungen der Formel (I) wie in irgendeinem der Ansprüche 1 bis 6 definiert in Form der Mischungen 9S1S,9S1R, **dadurch gekennzeichnet, daß** man eine Verbindung der Formel (I) der Einwirkung eines Mittels zur Racemat-Auftrennung des durch den Ring in Position 1 getragenen asymmetrischen Kohlenstoffs unterzieht, zur Herstellung der Verbindung der Formel (Iopt): 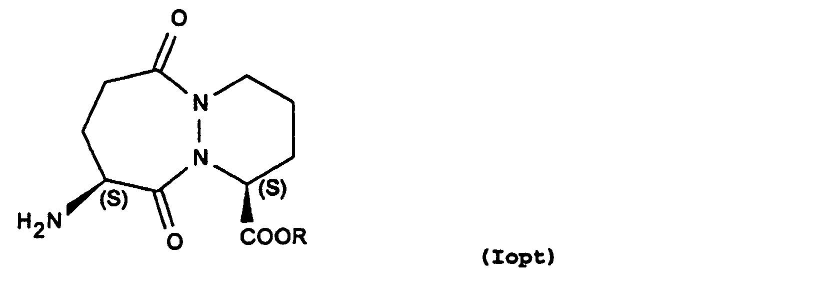 in der Form 9S1S, bei der die Aminfunktion frei oder geschützt ist und R wie in Anspruch 1 definiert ist.

10. Verwendung nach Ansprüch 9 der Verbindungen der Formel (IA) wie in irgendeinem der Ansprüche 2 bis 6 definiert zur Herstellung der Verbindung der Formel (IAopt) : 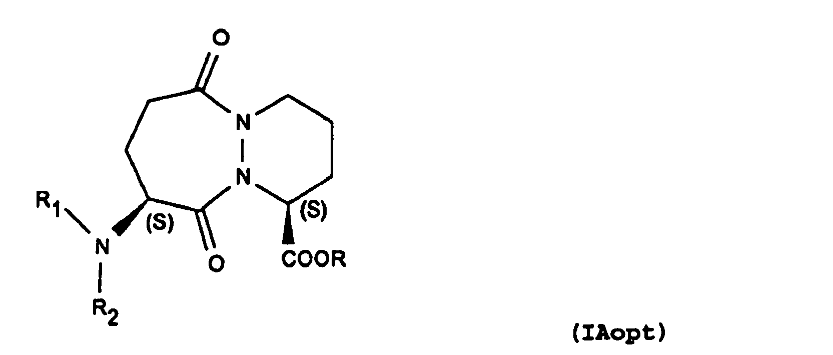 in der Form 9S1S, in der R, R₁ und R₂ ihre Definition wie in Anspruch 2 beibehalten.

11. Verwendung nach Anspruch 9 und 10, **dadurch gekennzeichnet, daß** R einen Rest Methyl darstellt.

12. Verwendung nach Anspruch 9, 10 oder 11, **dadurch gekennzeichnet, daß** die Aminfunktion in Form von Phthalimido geschützt ist.

13. Verwendung nach irgendeinem der Ansprüche 9 bis 12, **dadurch gekennzeichnet, daß** das Mittel zur Racemat-Auftrennung eine Base ist.

14. Verwendung nach Anspruch 13, **dadurch gekennzeichnet, daß** die starke Base ein Alkoholat von Alkali oder Erdalkali oder ein lithiumhaltiges Amin ist.

15. Verwendung nach irgendeinem der Ansprüche 9 bis 14, **dadurch gekennzeichnet, daß** das Ausgangsprodukt die racemische Mischung nach Anspruch 6 ist und das hergestellte Produkt
- (1S-cis)-9-(1,3-Dihydro-1,3-dioxo-2H-isoindol-2-yl)-octahydro-6,10-dioxo-6H-pyridazino[1,2-a]-[1,2]diazepin-1-carbonsäure-methylester ist.
